(19) Europäisches Patentamt
European Patent Office
Office européen des brevets

(11) Publication number: 0 070 185
B1

(12) **EUROPEAN PATENT SPECIFICATION**

(45) Date of publication of patent specification: **10.04.85**

(51) Int. Cl.$^4$: **C 07 D 213/55**

(21) Application number: **82303659.5**

(22) Date of filing: **13.07.82**

# (54) Preparation of lower alkyl 2-formyl-3-(6-methyl-3-pyridinyl)-propionates.

(30) Priority: **14.07.81 GB 8121641**

(43) Date of publication of application:
**19.01.83 Bulletin 83/03**

(45) Publication of the grant of the patent:
**10.04.85 Bulletin 85/15**

(84) Designated Contracting States:
**AT BE CH DE FR GB IT LI NL**

(56) References cited:
**US-A-3 437 676**
**US-A-3 952 034**
**US-A-4 153 795**
**US-A-4 234 588**

**CHEMICAL ABSTRACTS, vol. 85, 1976, page 486, no. 177018t, Columbus, Ohio, USA, Y. SUGI et al.: "The palladium-catalyzed carbonylation of styrene. Effects of phosphines on the products"**

**The file contains technical information submitted after the application was filed and not included in this specification**

(73) Proprietor: **SMITHKLINE BECKMAN CORPORATION**
**P.O. Box 7929 1 Franklin Plaza**
***Philadelphia* Pennsylvania 19101 (US)**

(72) Inventor: **Levinson, Sidney Harry**
**2508 Stoneybrook Lane**
**Drexel Hill Pennsylvania (US)**
Inventor: **Mendelson, Wilford Lee**
**592 General Learned Road**
**King of Prussia Pennsylvania (US)**
Inventor: **Morrell, Dennis Glen**
**1004 Shearwater Drive**
**Audubon Pennsylvania (US)**

(74) Representative: **Johnston, Walter Paul et al**
**Patent Department SMITH KLINE & FRENCH LABORATORIES LTD Mundells**
**Welwyn Garden City Hertfordshire AL7 1EY (GB)**

(56) References cited:
**JOURNAL OF ORGANOMETALLIC CHEMISTRY, no. 132, 1977, pages C26-C28, Lausanne, CH. G. CONSIGLIO: "Asymmetric hydrocarboxylation of olefins. IV. Influence of PPH3 in the asymmetric hydroxarboxylation catalyzed by ((-)-DIOP)PdC12"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 070 185 B1

Courier Press, Leamington Spa, England.

**Description**

This invention provides a chemical method for preparing lower alkyl 2-formyl-3-(3-pryidinyl)-propionates which are intermediates for preparing chemical compounds having histamine $H_2$-receptor antagonist activity whose structures are characterized by being 2-substituted amino-5-heterocyclylmethyl-4(1H)-pyrimidinones. See, for example, U.S. Patent No. 4,234,588. The method of this invention comprises two chemical reactions, first and most critical, a homogeneous catalytic hydrocarboxyalkylation of a 3-vinylpyridine to give a 3-(3-pyridinyl)propionic acid ester and then α-formylation of this ester.

Hydroxycarboxyalkylation of olefins by means of a catalytic condensation is a chemical process well known to the art, for example Wender et al., *Organic Synthesis via Metal Carbonyls* Vol. 2, page 233 (Wiley 1977) and U.S. Patent No. 3,437,676. The reaction is essentially the addition of carbon monoxide and an alcohol (or water) to a vinyl group. The catalytic hydrocarboxyalkylation of styrene has been reported to produce 95% of the 2-ester using a palladium-tin catalyst (U.S. Patent No. 3,952,034). Also, using diphosphine palladium complexes, a process for the hydrocarboxyalkylation of styrene to favor the production of the 3-ester has been reported, Y. Sugi et al., *Chemistry Letters,* 727—730 (1976) and Japanese Patent Application No. Sho 49—143454 (June 18, 1976). Many catalysts are also known to this art such as palladium halides bonded or not with various ligands such as phosphines, rhodium or ruthenium catalysts, cobalt carbonyls and others. Hydrocarboxyalkylation of 2-vinylpyridine using cobalt carbonyl ($Co_2(CO)_8$) with pyridine as ligand gave 3-esters, as reported by A. Madsuda et al., *Bull. Chem. Soc. Japan* 51:3016 (1978).

We have now found that with certain specific catalysts under homogenous conditions 6-methyl-3-vinylpyridine can by hydrocarboxyalkylated using carbon monoxide and a $C_{1-4}$ alkanol, especially either methanol or ethanol, to form a mixture of lower alkyl 3-(6-methyl-3-pyridinyl)propionate and 2-(6-methyl-3-pyridinyl)propionate, unexpextedly with a high proportion of the desired 3-isomer.

The method is carried out most conveniently by using one mole equivalent of the 6-methyl-3-vinyl-pyridine, 3—30 mole equivalents of $C_{1-4}$ alkanol and a catalytic quantity (1/4—1/3 mole %) of the palladium compound. When 3—6 mole equivalents of alkanol are used, the process is preferably carried out in an aromatic solvent which is chemically inert under the reaction conditions and in which the reactants are soluble. Examples of suitable solvents are the benzenoid solvents such as benzene, toluene, xylene, anisole, chlorobenzene and the like. Toluene is preferred. The selectivity to the 3-isomer vs. the 2-isomer is higher when aromatic solvents are used.

In addition unexpectedly enhanced yields are obtained when the hydrocarboxyalkylation reaction is carried out in the presence of one or more mole equivalents of an acid such as glacial acetic acid.

The palladium compound, which is a catalyst precursor, has the formula:

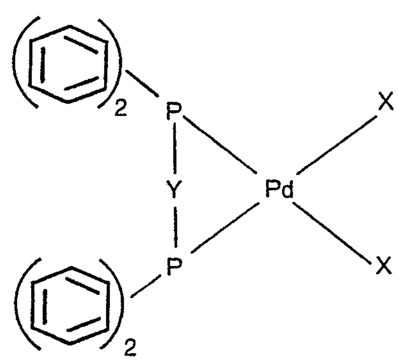

in which Y is $(CH_2)_n$, $CH_2CH_2\overset{+}{N}H_2CH_2CH_2Cl^-$,

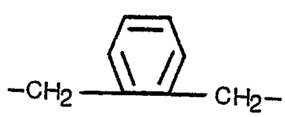

or

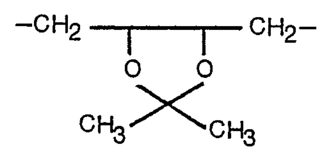

n is 3, 4 or 5; and
X is Cl, Br, $HSO_4$ or $NO_3$.

The phosphine ligand has 3, 4 or 5 atoms, particularly 4, separating the two diphenylphosphine groups. These palladium compounds, especially where Y is $CH_2CH_2NH_2^+CH_2CH_2$ $Cl^-$ may be bound to polymer systems by methods known to the art.

One skilled in the art will recognise that certain aromatic solvents such as 1,4-dichlorobenzene may

also associate with the catalyst as an additional ligand without adversely affecting the course of the reaction.

The method is most usefully carried out in an atmosphere of carbon monoxide charged to about 69—10350 KPa (10—1500 p.s.i.) preferably 344—2070 KPa (50—300 p.s.i.) at temperatures of from about 75—200° preferably 90—150°C until reaction is substantially complete such as from 8—12 hours. Generally pressures less than 2070 KPa (300 p.s.i.) are preferred.

The course of the first step of the reaction sequence of this invention is as follows expressed with preferred reactants.

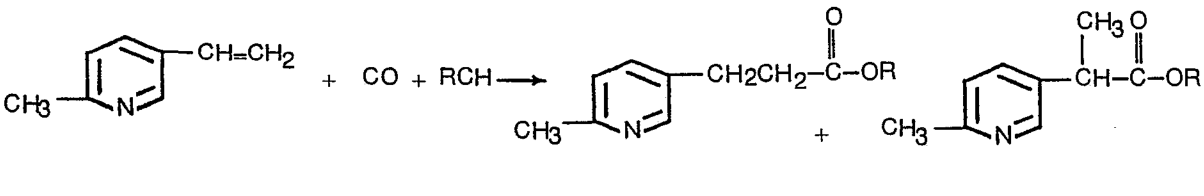

II III IV

in which R is $C_{1-4}$ alkyl.

The configuration necessary for the next step of this process is the 3-isomer of structure III above. Unexpectedly, the process of this invention provides a ratio of the 3-isomer to the 2-isomer of structure IV of 6—8 to 1 under preferred conditions. In addition to the hydrocarboxyalkylation products, III and IV, there are produced a 3-ethylpyridine and a 3-(3-pyridinyl)propionic acid as by-products.

The desired 3-(6-methyl-3-pyridinyl)propionate (III) can be isolated by standard chemical means. It has been found, however, that under simple distillation conditions the two isomeric products usually codistill especially the ethyl and methyl esters. However, a mixture of the isomers may be formylated and the formylated derivative of III being acidic can be separated using an aqueous base extraction.

As noted above, addition of one mole-equivalent or an excess of glacial acetic acid to the catalytic reaction mixture unexpectedly gives increased yields of the desired 3-(6-methyl-3-pyridinyl)propionate.

A number of hydrocarboxyalkylation catalysts commonly used in the prior art have either not worked or have given poor yields of the desired products in our hands such as cobalt carbonyl, bis-benzonitrile palladium dichloride, bis-triphenylphosphine palladium dichloride, bis-triphenylphosphine palladium dichloride-stannous chloride. At this point, therefore, it seems that the character of the ligand in the catalyst is critical in determining selectivity for the 3-isomer.

The second chemical step of the process of this invention comprises α-formylation of the 3-(6-methyl-3-pyridinyl)propionate ester.

The reaction is illustrated as follows (formylation of compound III to give compound V):

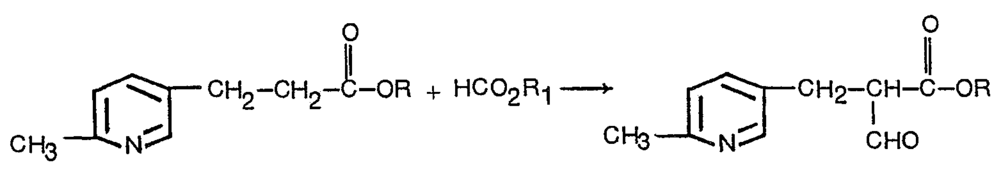

III V

in which R is as described above and $R_1$ is methyl or ethyl.

The formylation of III using ethyl formate under somewhat different reaction conditions is disclosed in U.S. Patent No. 4,234,588, for example in Example 3.

Most readily the formylation is carried out by reacting the ester with an excess of methyl or ethyl formate in the presence of an acid binding agent such as a sodium or potassium lower alkoxide of 1—6 carbons preferably methoxide or ethoxide. As solvent, one may use any organic solvent, inert under the reaction conditions, in which the reactants are soluble, such as an aromatic solvent such as benzene, toluene, xylene, anisole or the like or an ether such as tetrahydrofuran. The course of the reaction is monitored while the reaction is allowed to go to completion either at ambient temperature or elevated temperature such as up to 50—60°. At room temperature reaction time may run from about 8—72 hours.

The source of ester starting material may be pure propionate ester III or a mixture of the 2 and 3-isomers as obtained directly from the catalytic hydrocarboxyalkylation reaction described above.

There is a large quantity of carbon monoxide gas evolved during the reaction using methyl formate due to decomposition of the formate. Yields of 50—90% based on the ester starting material are realized by carrying the reaction out under preferred confined conditions either in a sealed reaction vessel or, for quantities above about 1/4 mole, in an autoclave.

Also, it has been found that when R and $R_1$ are not identical transesterification occurs which gives a

mixture of 3-(6-methyl-3-pyridinyl)-2-formylpropionate esters. Therefore, it is preferred that R and $R_1$ be the same and also that the same alkyl group be present in the alkoxide acid binding agent.

The desired product is isolated by conventional chemical methods.

Other α-formylation methods can also be used such as by reacting the propionate ester with carbon monoxide under catalytic conditions or in the presence of a sodium or potassium lower alkoxide as known to the art.

The α-formyl product forms a water soluble sodium or potassium salt in its enolic form.

The following examples are designed to teach the practice of this invention. All temperatures are in degrees Centigrade.

Example 1

A mixture of 180 g (1.5 m) of 2-methyl-5-vinyl-pyridine, 200 g (6.25 m) of methanol, 100 g (1.67 m) of glacial acetic acid, a 3.4 g (0.0056 m) of 1,4-bis(diphenylphosphino)butane palladium dichloride and 2600 ml of toluene was charged into a one gallon stirred autoclave. The container was pressurized to 1035 KPa (150 p.s.i) with carbon monoxide. The mixture was heated to 120°C with agitation for 10 hours. The autoclave was then vented. The contents were placed on a rotary evaporator to obtain an oil which was distilled to obtain 135 g (100°, 0.4 mmHg) of product containing 87% (44% yield) of methyl 3-(6-methyl-3-pyridinyl)propionate and 13% (6.5% yield) of methyl 2-(6-methyl-3-pyridinyl)propionate (analysis by gas chromatography).

Example 2

A mixture of 45 g (0.25 m) of methyl 3-(6-methyl-3-pyridinyl)propionate in the form of a mixture with its 2-isomer, 155.2 g (2.59 m) of methyl formate and 270 ml of toluene was placed in a 1 1. stirring autoclave. Then 34 g (0.63 m) of sodium methoxide was added slowly. The sealed autoclave was heated to 35—40° for a total of 16 hours. The light yellow mixture was removed from the reactor for workup.

Water in several portions was added to the toluene reaction mixture. The water extracts were combined and adjusted to pH 6.5 with concentrated sulfuric acid to separate a white solid. The mixture was chilled and filtered to give 44.9 g (86%) of crude methyl 3-(6-methyl-3-pyridinyl)-2-formylpropionate. This material may be used in crude form. It was purified further by dissolving in hot chloroform, filtering and diluting with 2 volumes of n-heptane. Cooling separated the purified ester. The ester has a melting point of 145.5—147°.

Anal. Calcd. for $C_{11}H_{13}NO_3$ 1/8 $H_2O$: C, 63.07; H, 6.38; N, 6,69. Found: C, 62.90, 63.07; H, 6.24, 6.25; N, 6.66, 6.72.

Example 3

Sodium (2.66 g, 0.11 m) was added slowly to a mixture of absolute ethanol (50 ml) and toluene (65 ml). The mixture was then heated until the sodium had all reacted and further to evaporate excess ethanol. The mixture was then cooled while 9.26 g (0.048 m) of ethyl 3-(6-methyl-3-pyridinyl)propionate and 8.58 g (0.116 m) of ethyl formate were added maintaining a temperature less than 15°. The mixture was then stirred at ambient temperature for 48 hours.

The mixture was cooled and crushed ice added. The pH was adjusted to pH 7 with 10N sulfuric acid to separate 7.9 g (72%) of ethyl 3-(6-methyl-3-pyridinyl)-2-formylpropionate. After purification by recrystallization and drying the melting point of the ethyl ester was 141—143°C.

Example 4

A slurry of 13.56 g (0.251 m) of sodium methoxide in 100 ml of toluene was chilled in ice water while 18.6 g (0.104 m) of methyl 3-(6-methyl-3-pyridinyl)propionate and 18.52 (0.251 m) of ethyl formate were added. The mixture was stirred at ambient temperature 60 hours. The reaction mixture was worked up as above to give 11.26 g (60%) of solid which by nuclear magnetic resonance analysis was a mixture of methyl and ethyl 3-(6-methyl-3-pyridinyl)-2-formylpropionate.

Example 5

By the procedure of Example 1, the following results were obtained using, in place of 1,4-bis(diphenylphosphine)butane, the following palladium compounds:

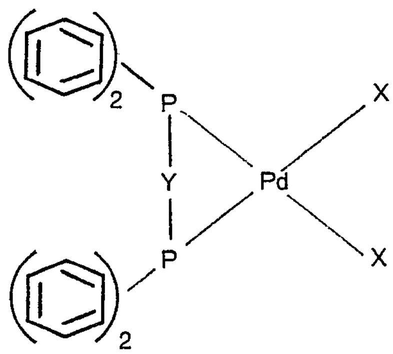

| | | % Yield | |
|---|---|---|---|
| Y | X | 3-ester | 2-ester |
| $-(CH_2)_3-$ | Cl | 37 | 8 |
| $-(CH_2)_5-$ | Cl | 35 | 4.3 |
| $-CH_2CH_2\overset{+}{N}H_2CH_2CH_2\ Cl^-$ (methanol solvent) | Cl | 71 | 15 |
| 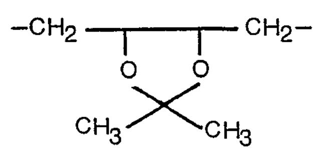 | Cl | 40 | 5.2 |
| $-(CH_2)_4-$ | Br | 42 | 5.1 |
| $-(CH_2)_4-$ (methanol solvent) | $HSO_4$ | 71 | 17 |
| $-(CH_2)_4-$ (methanol solvent) | $NO_3$ | 59 | 16 |

The palladium compounds are either known to the art or are prepared by known methods, for example by reaction of the phosphines with bis(benzonitrile)palladium dichloride, Sugi et al., *Chemistry Letters*, 727—730 (1976).

Example 6

By the procedure of Example 1, using the following lower alkanols in place of methanol, the following results were obtained:

| | | % Yield | |
|---|---|---|---|
| lower alkanol | ester | 3-ester | 2-ester |
| ethanol | ethyl | 46.8 | 4.3 |
| n-propanol | n-propyl | 32 | 2.6 |
| n-butanol | n-butyl | 31 | 2.6 |

**Claims**

1. A process for preparing a compound of the formula:

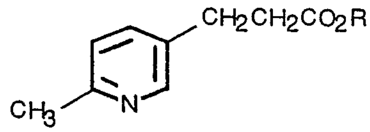

wherein R is $C_{1-4}$alkyl, which comprises reacting:

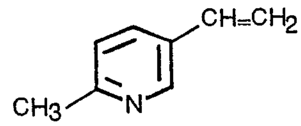

with carbon monoxide and a $C_{1-4}$alkanol under homogenous catalytic conditions in the presence of one or more mole equivalents of glacial acetic acid (based on the vinylpyridine) and in the presence of a catalyst precursor of the structure:

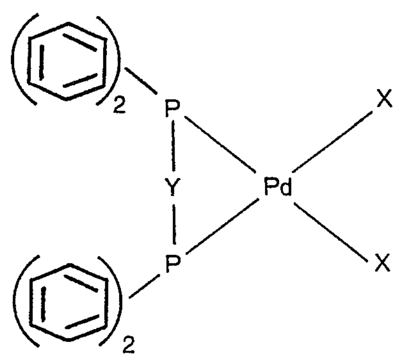

in which Y is $(CH_2)_n$, $CH_2CH_2\overset{+}{N}H_2CH_2CH_2\ Cl^-$,

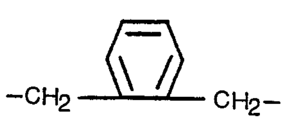

or

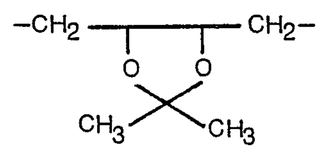

n is 3, 4 or 5; and
X is Cl, Br, $HSO_4$ or $NO_3$.

2. A process according to claim 1 in which n is 4.

3. A process according to claim 1 in which n is 4, the pressure of reaction is from 344—2070 KPa (50—300 p.s.i.), the temperature is from 90—150°C and the alkanol is ethanol or methanol.

4. A process according to any one of claims 1 to 3 in which toluene is present as a solvent.

5. A process according to any one of claims 1 to 4 in which the $C_{1-4}$alkyl 3-(6-methyl-3-pyridinyl)-propionate is then α-formylated.

6. A process according to claim 5 in which the formylating reagent is methyl formate in the case of methyl 3-(6-methyl-3-pyridinyl)propionate and ethyl formate in the case of ethyl 3-(6-methyl-3-pyridinyl)-propionate.

7. A process according to claim 5 in which the formylation is carried out by reacting methyl formate and methyl 3-(6-methyl-pyridinyl)propionate in the presence of sodium or potassium methoxide in a confined reaction vessel.

8. A process according to claim 5 in which the formylation is carried out using a crude mixture of 2- and 3-propionate isomers.

**Patentansprüche**

1. Verfahren zur Herstellung einer Verbindung der Formel

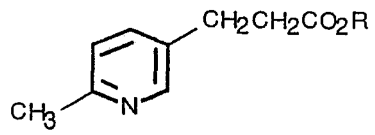

in der R einen $C_{1-4}$-Alkylrest darstellt, dadurch gekennzeichnet, daß man die Verbindung

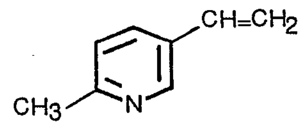

mit Kohlenmonoxid und einem $C_{1-4}$-Alkanol unter homogenen katalytischen Bedingungen in Gegenwart eines oder mehrerer Moläquivalente Eisessig (bezogen auf das Vinylpyridin) und in Gegenwart eines Katalysator-Vorläufers der Struktur umsetzt:

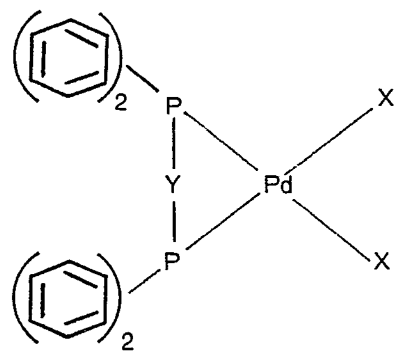

in der Y $(CH_2)_n$, $CH_2CH_2\overset{+}{N}H_2CH_2CH_2\ Cl^-$,

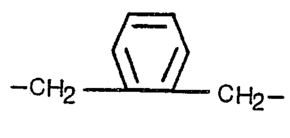

oder

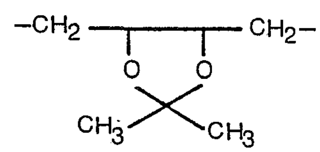

darstellt, n den Wert 3, 4 oder 5 hat und X Cl, Br, $HSO_4$ oder $NO_3$ ist.

2. Verfahren nach Anspruch 1, wobei n den Wert 4 hat.

3. Varfahren nach Anspruch 1, wobei n den Wert 4 hat, der Reaktionsdruck 344 bis 2070 KPa (50—300 p.s.i.) beträgt, die Temperatur 90 bis 150°C beträgt und der Alkanol Äthanol oder Methanol ist.

4. Verfahren nach einem der Ansprüche 1 bis 3, wobei Toluol als Lösungsmittel zugegen ist.

5. Verfahren nach einem der Ansprüche 1 bis 4, wobei der 3-(6-Methyl-3-pyridinyl)-propionsäure-$C_{1-4}$-alkylester dann α-formyliert wird.

6. Verfahren nach Anspruch 5, wobei das Formylierungsreagens im Fall von 3-(6-Methyl-3-pyridinyl)-propionsäure-methylester Ameisensäuremethylester und im Fall von 3-(6-Methyl-3-pyridinyl)-propionsäure-äthylester Ameisensäureäthylester ist.

7. Verfahren nach Anspruch 5, wobei die Formylierung durch Umsetzung von Ameisensäuremethylester und 3-(6-Methyl-3-pyridinyl)-propionsäuremethylester in Gegenwart von Natrium- oder Kaliummethoxid in einem engen Reaktionsgefäß durchgeführt wird.

8. Verfahren nach Anspruch 5, wobie die Formylierung unter Verwendung eines rohen Gemisches aus den 2- und 3-Propionsäureesterisomeren durchgeführt wird.

**Revendications**

1. Un procédé pour la préparation d'un composé de la formule:

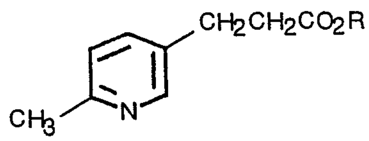

dans laquelle R est $C_{1\text{-}4}$ alcoyle, qui comprend la réaction:

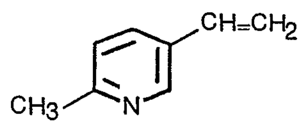

avec de l'oxyde de carbone et un alcanol $C_{1-4}$ dans des conditions catalytiques homogènes en présence d'un ou plusieurs équivalents mole d'acide acétique glacial (se basant sur la vinylpyridine) et en présence d'un précurseur catalyste de la structure:

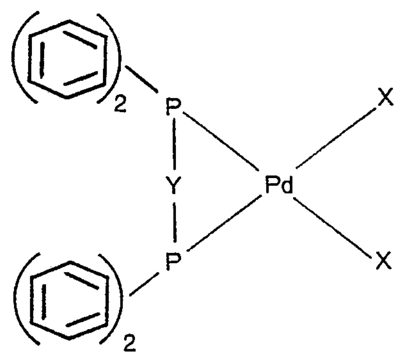

dans laquelle Y est $(CH_2)_n$, $CH_2CH_2\overset{+}{N}H_2CH_2CH_2\ Cl^-$,

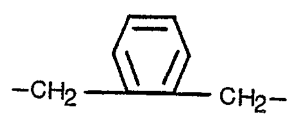

ou

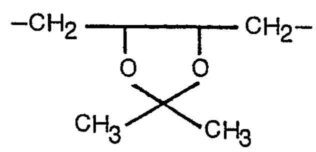

n est 3, 4 ou 5; et
X est Cl, Br, $HSO_4$ ou $NO_3$.

2. Un procédé selon la revendication 1, dans lequel n est 4.

3. Un procédé selon la revendication 1, dans lequel n est 4, la pression de la réaction se situe à partir de 344—2070 KPa (50—300 p.s.i.), la température est à partir de 90—150°C et l'alcanol est éthanol ou méthanol.

4. Un procédé selon n'importe laquelle des revendications de 1 à 3, dans lequel le toluène est présent en tant que solvant.

5. Un procédé selon n'importe laquelle des revendications de 1 à 4, dans lequel le $C_{1\text{-}4}$ alcoyle 3-(6-méthyle-3-pyridinyle) propionate est ensuite formylé-alpha.

6. Un procédé selon la revendication 5 dans lequel le réactant de formylation est du formate méthyle dans le cas de méthyle 3-(6-méthyle-3-pyridinyle) propionate et du formate éthyle dans le cas d'éthyle 3-(6-méthyle-3-pyridinyle) propionate.

7. Un procédé selon la revendication 5 dans lequel on effectue la formylation en faisant réagir du formate méthyle et du méthyle 3-(6-méthyle-3-pyridinyle) propionate en présence de méthoxyde de sodium ou de potassium dans un récipient de réaction restreint.

8. Un procédé selon la revendication 5, dans lequel on effectue la formylation en se servant d'un mélange brut d'isomères de propionate 2- et 3.